**Europäisches Patentamt**

**European Patent Office**

**Office européen des brevets**

(11) Veröffentlichungsnummer: **0 045 916**

**A1**

(12) EUROPÄISCHE PATENTANMELDUNG

(21) Anmeldenummer: **81106035.9**

(22) Anmeldetag: **31.07.81**

(51) Int. Cl.³: **A 61 B 5/10**

(30) Priorität: **11.08.80 US 176700**

(43) Veröffentlichungstag der Anmeldung:
**17.02.82 Patentblatt 82/7**

(84) Benannte Vertragsstaaten:
**AT BE CH DE FR GB IT LI NL SE**

(71) Anmelder: **SIEMENS AKTIENGESELLSCHAFT Berlin
und München
Postfach 22 02 61
D-8000 München 22(DE)**

(72) Erfinder: **Chiu, Ming-Yee, Dr.
78 Boothby Drive
Mt. Laurel, New Jersey 08054(US)**

(54) Fingerabdrucksensor zum Erzeugen eines dem Fingerabdruck entsprechenden Ausgangssignals.

㉗ Es wird ein Fingerabdrucksensor beschrieben, der eine Kontaktplatte (14) aufweist, die aus einem transparenten Material gebildet ist. Eine Seite der Kontaktplatte (14) weist eine Kontaktfläche (20) auf. Wenn ein Finger (2) auf die Kontaktfläche (20) gedrückt wird, ändert diese ihre Struktur entsprechend dem topografischen Relief des Fingers (2). Die andere Seite der Kontaktplatte (14) ist mit einem Substrat (22) bedeckt, welches einen vergleichsweise niedrigen Brechungsindex aufweist. Ein Lichteinkoppler, der aus einem Prisma (12) oder einem Gitterkoppler bestehen kann, ist zum Einkoppeln von Licht in die Kontaktplatte (14) vorgesehen. Das eingekoppelte Licht wird in der Kontaktplatte (14) total reflektiert. Vom Fingerabdruck auf der Kontaktfläche (20) reflektiertes Licht wird einem Fotodetektor (36, 38) zugeführt, und dieser mißt die Intensitätsverteilung des auftreffenden Lichts.

FIG. 1

Croydon Printing Company Ltd.

SIEMENS AKTIENGESELLSCHAFT          Unser Zeichen
Berlin und München                  VPA 80 P 8234


Fingerabdrucksensor zum Erzeugen eines dem Fingerabdruck entsprechenden Ausgangssignals


Die Erfindung bezieht sich auf einen Fingerabdrucksensor zum Erzeugen eines dem topografischen Relief
eines zu untersuchenden Fingers entsprechenden Ausgangssignals.


Systeme zum Identifizieren des Abdrucks eines auf eine
Kontaktfläche aufgedrückten Fingers sind bekannt.


Beispielsweise geht aus der US-PS 4 053 228 ein
Fingeridentifizierungsapparat hervor, der eine transparente Glasplatte aufweist, die als eine Kontaktfläche
oder als Fingerabdruckleser dient. Ein Fingerabdruck
wird dadurch ausgebildet, daß der zu untersuchende
Finger auf die Rückfläche der Glasplatte gedrückt
und in einer vorbestimmten Stellung stillgehalten
wird. Der Fingerabdruck wird durch einen die Vorderfläche der Glasplatte durchstrahlenden Lichtstrahl
abgefragt. Der abfragende Strahl wird an der Rückfläche teilweise reflektiert, so daß ein Signalstrahl
entsteht, der die Fingerabdruckinformation trägt.
Der reflektierte Signalstrahl wird dann mit einem
Hologramm des gleichen Fingerabdrucks korreliert, so
daß die Identifizierung der Person erhalten wird.


Aus der US-PS 4 120 585 geht ein anderes System zur
Identifizierung eines Fingerabdrucks hervor. Dieses
System weist ein nachgiebiges oder biegsames optisches
Prisma als Fingerabdrucksensor auf. Die Basis des
Prismas wird von dem Finger der zu untersuchenden
Ed 1 Sti/31.7.81

Person berührt. Das nachgiebige Prisma verfomt sich unter dem angewendeten Druck., Es reflektiert im Inneren einen abtastenden Lichtstrahl in Richtung einer fotoempfindlichen Vorrichtung, die aktiviert wird. Die fotoempfindliche Vorrichtung aktiviert ihrerseits weitere optische Komponenten des Fingerabdruckidentifizierungssystems. Ein Fingerabdruckleser wird auf das Muster der Erhöhungen und Vertiefungen des Fingerabdrucks einer zu untersuchenden Person hin überprüft. Es sei darauf hingewiesen, daß bei diesem bekannten System die Prismenfläche als Ganzes gebogen wird. Der Finger drückt in die Fläche kein Muster in der Weise ein, daß eine Konfiguration des topografischen Reliefs des Fingers entsteht.

Die bekannten Fingerabdrucksysteme erfordern eine ziemlich aufwendige Technologie. Ein Fingerabdruckidentifizierungssystem sollte jedoch leicht und billig zusammengebaut werden können und gleichzeitig sollte es eine hohe Bildqualität des Fingerabdrucks liefern.

Die Aufgabe der Erfindung besteht darin, einen Fingerabdrucksensor der eingangs genannten Art anzugeben, der leicht zusammengebaut werden kann und billig ist, und der Fingerabdruckbilder hoher Qualität erzeugt.

Diese Aufgabe wird durch die im kennzeichnenden Teil des Patentanspruchs 1 angegebenen Merkmale gelöst.

Durch diese Lösung ist ein Fingerabdrucksensor geschaffen, der die Fingerabdruckinformation eines berührenden Fingers in ein elektrisches Ausgangssignal umwandelt. Das elektrische Ausgangssignal stellt die in dem Fingerabdruck enthaltene Information dar und kann zur Weiterverarbeitung in einen Rechner eingelesen werden.

Der Sensor weist eine Sensorplatte kleiner Dicke
auf.

Er verarbeitet einen hohen Anteil von aus einer zur
Beleuchtung eines Fingerabdrucks vorgesehenen Lichtquelle ausgesandten Lichts. Sein Wirkungsgrad ist
hoch.

Ein weiterer Vorteil des Sensors liegt darin, daß
er eine Lichteinkoppelvorrichtung zum Einkoppeln
von Licht in eine Sensorplatte verwendet.

In Zusammenfassung weist ein erfindungsgemäßer
Fingerabdrucksensor zum Umwandeln der Fingerabdruckinformation eines berührenden Fingers in
ein elektrisches Ausgangssignal eine aus einem
transparenten und elastischen Material gebildete
Kontaktplatte auf. Diese Kontaktplatte, die dünn
sein kann, weist zwei parallele Seiten auf. Die
erste Seite weist eine Lichtaufnahmefläche zur Aufnahme von Licht und eine Kontaktfläche auf, auf die
von dem zu überprüfenden Finger ein Kontaktdruck
auszuüben ist. Wenn der Finger auf die Kontaktfläche gepreßt wird, wird deren glatte Struktur
entsprechend dem Muster des Fingerabdrucks verändert, weil die elastischen Eigenschaften des
Materials so gewählt sind. Nach Fortnahme des
Fingers von der Kontaktfläche nimmt diese wieder
ihre frühere Struktur ein, d.h. sie wird wieder
glatt und eben. Die zweite Seite der Kontaktplatte ist von einem Substrat bedeckt. Der Brechungsindex der Kontaktplatte ist größer als der
Brechungsindex des Substrats.

Der Fingerabdrucksensor weist auch einen der Lichtaufnahmefläche zugeordneten oder damit verbundenen optischen Koppler auf. Dieser optische Koppler kann aus einem Prisma und/oder aus einem Gitterkoppler bestehen.

Der Fingerabdrucksensor weist auch eine Lichtquelle auf. Die Lichtquelle führt einen ersten Lichtstrahl über den optischen Koppler und über die Lichtaufnahmefläche dem Inneren der Kontaktplatte zu. Dort findet Totalreflexion statt. Die Kontaktfläche wird deshalb von dem zwischen den beiden Seiten der Kontaktplatte sich ausbreitenden Licht berührt. In Gegenwart eines andrückenden Fingers reflektiert die Kontaktfläche einen Lichtstrahl durch die Kontaktplatte und durch das Substrat. Das reflektierte Licht ist mit dem Fingerabdruckmuster des Fingers moduliert.

Der Fingerabdrucksensor weist des weiteren ein optisches System und einen Fotodetektor auf. Der Fotodetektor weist einen lichtempfindlichen Bereich zum Messen der Verteilung des auf ihn treffenden Lichts auf. Das optische System führt das von der Kontaktfläche reflektierte, modulierte Licht dem lichtempfindlichen Bereich des Fotodetektors zu.

Im optischen Strahlengang zwischen dem optischen System und dem lichtempfindlichen Bereich des Fotodetektors ist eine Blende angeordnet, die einen Teil des reflektierten Lichts ausblendet. Dieser Teil ist in die Brennebene des optischen Systems fokussiert.

Der Fingerabdrucksensor weist zusätzlich einen dem Fotodetektor zugeordneten Ausgang auf, aus dem das elektrische Ausgangssignal erhalten werden kann.

Das elastische Material, aus welchem die Kontaktfläche des Kontaktkörpers gebildet ist, kann ein Polymer sein. Insbesondere hat sich Silikonkautschuk oder

-5-        VPA 80 P 8234

-gummi als brauchbar erwiesen. Aber auch andere
Elastomere können verwendet werden.

Bevorzugte Ausführungsformen der Erfindung sind in
den Figuren dargestellt und werden in der folgenden
Beschreibung näher erläutert. Von den Figuren zeigen:

Figur 1 eine Ausführungsform eines Fingerabdrucksensors,
        der einen Prismenkoppler aufweist,

Figur 2 einen teilweisen Querschnitt durch eine Ausführungsform eines Fingerabdrucksensors, der
        einen Gitterkoppler aufweist,

Figur 3 eine Draufsicht auf die Ausführungsform des
        Fingerabdrucksensors nach Figur 2,

Figur 4 einen  Querschnitt durch eine Ausführungsform
        nach Figur 2, bei welcher der Gitterkoppler
        ein Phasengitter aufweist,

Figur 5 einen Querschnitt durch eine andere Ausführungsform gemäß Figur 2 mit einem ein anderes Phasengitter aufweisenden Gitterkoppler,

Figur 6 einen Querschnitt durch eine Ausführungsform
        eines Fingerabdrucksensors, der auf beiden
        Seiten Prismenkoppler aufweist,

Figur 7 eine Draufsicht auf eine Ausführungsform eines
        Fingerabdrucksensors, der vier Lichtkoppler
        aufweist, und

Figur 8 eine Draufsicht auf eine Ausführungsform eines
        Fingerabdrucksensors, der acht Lichtkoppler
        aufweist.

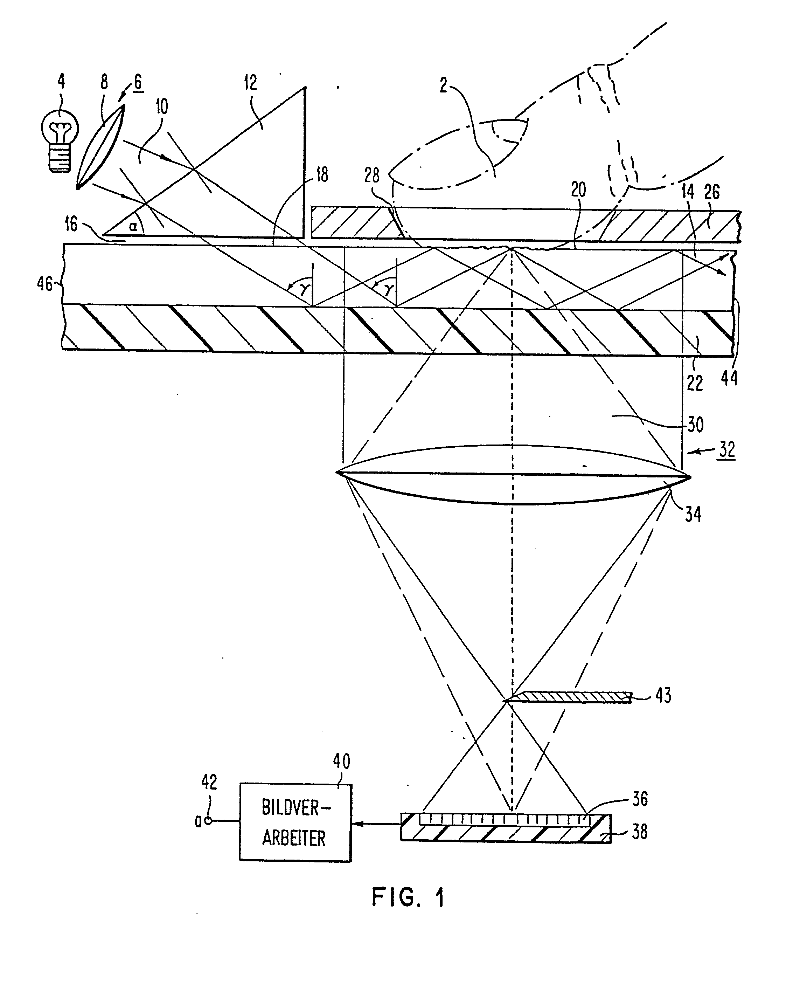

In der Figur 1 ist ein Fingerabdrucksensor zum Erzeugen eines dem topografischen Relief oder Muster eines zu untersuchenden Fingers 2 entsprechenden Ausgangssignals $\underline{a}$ dargestellt.

Der Fingerabdrucksensor weist eine Lichtquelle 4 auf, die einen Lichtstrahl zum Abtasten des Fingerabdrucks aussendet. Die Lichtquelle 4 kann eine Weißlichtquelle sein, beispielsweise eine reguläre Glühlampe. In der vorliegenden Ausführungsform gemäß Figur 1 besteht die Lichtquelle 4 aus einer kleinen Glühlampe. Die Lichtquelle 4 kann auch eine lichtemittierende Diode (LED) oder ein Halbleiterlaser sein, der Licht einer speziellen Wellenlänge aussendet. Die Wellenlänge des Lichts kann im infraroten Bereich des Spektrums liegen.

Das Licht aus der Lichtquelle 4 wird von einem ersten optischen System $\underline{6}$, das eine Kollimatorlinse 8 aufweist, gebündelt. Das optische System $\underline{6}$ führt einen parallelen Lichtstrahl 10 einem Prisma 12 zu. Wenn eine Lichtquelle 4 benutzt wird, die klein und weit entfernt ist, kann das erste optische System $\underline{6}$ fortgelassen werden.

Das Prisma 12 kann aus einem Glasprisma bestehen. Die Basisfläche und eine der beiden Seitenflächen des Prismas schließen einen Winkel $\alpha$ zwischen sich ein, der kleiner als 90° ist. Die Seitenfläche ist nahe der Oberseite einer dünnen Sensor- oder Kontaktplatte 14 und parallel dazu angeordnet. Die Seitenfläche des Prismas 12 ist von der Oberseite der Kontaktplatte 14 durch einen schmalen Spalt 16 getrennt. Der Spalt 16, dessen Breite bei-

-7-        VPA 80 P 8234

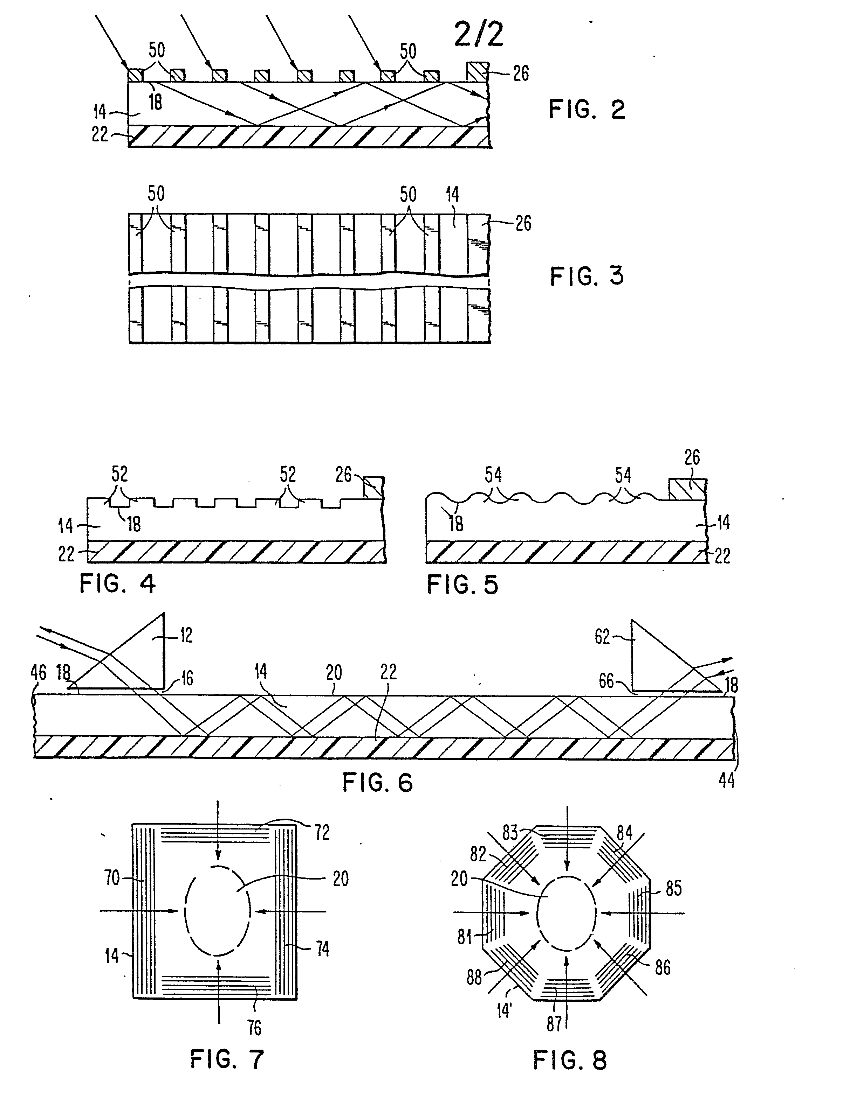

spielsweise 100 nm (= 1000 Ångström) beträgt, ist
ein Luftspalt oder enthält ein transparentes Medium
eines niedrigen Brechungsindexes. Das Prisma 12
bildet deshalb einen effektiven Lichteinkoppler.
Derartige Koppler sind in Zusammenhang mit dem
Einkoppeln von Licht in oder aus einem dünnen Film
bekannt (siehe Proc. IEEE, Vol. 62, Nr. 8, August
1974, S. 1044-1060, insbesondere Figur 8).

Die Kontaktplatte 14 weist eine plane Oberseite und
eine plane Unterseite auf. Beide Seiten oder Seitenflächen sind parallel zueinander. Die Oberseite weist
zwei verschiedene Bereiche auf, eine Lichtaufnahmefläche 18 und eine Kontaktfläche 20. Die Lichtaufnahmefläche 18 nimmt Licht aus dem Prisma 12 auf,
wenn die Lichtquelle 4 eingeschaltet ist, und die
Kontaktfläche 20 nimmt einen Kontaktdruck auf,
wenn der Abdruck des Fingers 2 untersucht wird.
Die Lichtaufnahmefläche 18 und die Kontaktfläche 20
können durch eine Stufe voneinander getrennt sein,
d.h. die Kontaktfläche 20 kann beispielsweise
gegenüber der Lichtaufnahmefläche 18 höher liegen.

An die Unterseite der Kontaktplatte 14 ist ein
Substrat oder eine Trägerplatte 22 angebracht,
die ebenfalls parallele Seiten aufweist. Zwischen
den beiden Platten 14 und 22 ist kein Spalt vorhanden. Der Brechungsindex des Substrats 22 ist
kleiner als der Brechungsindex der Kontaktplatte
14. Der Einfallswinkel $\psi$ des in der Kontaktplatte
sich vom Prisma 12 her ausbreitenden Lichts, der
in Bezug auf die Flächennormale der Unterseite
der Kontaktplatte 14 gemessen ist, ist größer als
der Totalreflexionswinkel gewählt.

-8-    VPA 80 P 8234

Der einfallende Lichtstrahl 10 durchstrahlt das Prisma 12, koppelt dann durch den Spalt 16 in die Kontaktplatte 14 über und erreicht die Oberseite des Substrats 22, wo Totalreflexion stattfindet. Von dort breitet sich das Licht in der Kontaktplatte 14 nach rechts und auch nach oben aus und trifft auf die Kontaktfläche 14. Die Kontaktplatte 14 kann als ein Lichtwellenleiter aufgefaßt werden.

Die Kontaktplatte 14 ist aus einem transparenten elastischen Material gebildet. Dieses elastische Material kann ein elastomerisches Material, beispielsweise ein Polymer sein. Vorzugsweise besteht das Material der Kontaktplatte 14 aus Silikonkautschuk oder -gummi. Das elastische Material kann die Oberseite der Trägerplatte 22 direkt überziehen. Wenn der Finger 2 die Kontaktplatte 14 nicht berührt, ist die Kontaktfläche 20 glatt und eben. Wenn jedoch der Finger 2 auf die Kontaktfläche 20 gedrückt wird, ändert sich die Struktur der Kontaktfläche 20. Sie enthält jetzt ein Muster, welches dem topografischen Relief der Erhöhungen und Vertiefungen oder Rippen Furchen des Fingerabdrucks entspricht.

Eine mit einer Öffnung 28 versehene Führungsplatte 26 kann dazu vorgesehen sein, den Finger 2 auf die Kontaktfläche 20 zu lenken, von wo aus der Fingerabdruck mittels einer optischen Einrichtung ermittelt werden kann. Zwischen der Führungsplatte 26 und der Kontaktplatte 14 kann eine lichtreflektierende Schicht oder ein entsprechender Überzug angeordnet sein.

Die Trägerplatte 22 soll der Plattenkonstruktion 14, 22 ausreichende Starrheit und Festigkeit verleihen.

Beides ist erwünscht, wenn der Finger 2 auf die Kontaktfläche 20 gedrückt wird. Die Trägerplatte 22 kann aus einem transparenten Kunststoff gebildet sein, der einen niedrigen Brechungsindex aufweist.

Wie schon erwähnt, durchstrahlt das Licht aus der Lichtquelle 4 das Prisma 12 und tritt in die Kontaktplatte 14 ein. Schließlich trifft es auf die Kontaktfläche 20. Wenn die Kontaktfläche 20 von der Haut des Fingers 2 besetzt ist, findet teilweise eine Spiegelreflexion statt.

Das von der Kontaktfläche 20 reflektierte Licht ist in der Figur 1 teilweise in durchgezogenen Linien und teilweise in gestrichelten Linien angedeutet. Eine Teil des reflektierten Lichtstrahls 30, der gestrichelt dargestellt ist, ist mit der topologischen Struktur des Fingers 2 räumlich moduliert. Der andere in durchgezogenen Linien dargestellte Anteil stellt eine Art Gleichlichthintergrund dar.

Der Lichtstrahl 30 trägt die Information über den Fingerabdruck. Er breitet sich nach unten aus und durchstrahlt ein zweites optisches System 32, das eine Abbildungslinse 34 oder ein entsprechendes Linsensystem aufweist.

Schließlich trifft der modulierte Lichtstrahl 30 den lichtempfindlichen Bereich 36 eines Fotodetektors 38. Der Fotodetektor 38 mißt die Verteilung des auf den lichtempfindlichen Bereich 36 auftreffenden Lichts. Dieser Bereich 36 kann aus einer Anordnung einzelner fotoempfindlicher Elemente bestehen. Die Ausgangssignale dieser Elemente werden einer digitalen Bildverarbeitungsvorrichtung 40 zugeführt, die in den Fotodetektor 38 eingebaut sein kann.

Vom Ausgang 42 dieser Vorrichtung 40 kann das elektrische Ausgangssignal a entnommen werden. Dieses Signal a kann gespeichert und weiter verarbeitet werden.

In Bezug auf die Abbildungslinse 34 des zweiten optischen Systems 32 ist der Fotodetektor 38 so angeordnet, daß das Bild des Fingerabdrucks scharf auf seinen lichtempfindlichen Bereich 36 abgebildet wird. Dies ist durch die gestrichelten Linien angedeutet, die sich auf dem Bereich 36 schneiden. Zwischen der Abbildungslinse 34 und dem lichtempfindlichen Bereich 36 ist eine Blendenvorrichtung 43 angeordnet, die aus einer Schneide, beispielsweise einer Messerschneide oder einer Rasierklinge bestehen kann. Die Blendenvorrichtung 43 ist so angeordnet, daß sie den reflektierten Lichtstrahl 30 teilweise ausblendet. Insbesondere ist sie so angeordnet, daß ihre Spitze oder Schneide das Bild der glühenden, insbesondere weißglühenden Lichtquelle 4 oder das von der Kontaktfläche 20 parallel zur optischen Achse des Systems 32 sich ausbreitende Licht ausblendet. Dieser Lichtanteil wird auf den lichtempfindlichen Bereich 36 nicht scharf abgebildet und stellt das bereits erwähnte Gleichlichthintergrund-Signal dar. Die Blendenvorrichtung 43 kann in der Brennebene der Abbildungslinse 34 angeordnet sein. Messerschneiden oder Raumfilter werden im Zusammenhang mit der sogenannten Schlierenmethode (siehe OPTICS, Febr. 1979, S. 478-481) verwendet.

Der Fotodetektor 38 kann eine Standard-Videokamera (Vidikonkamera) sein. Eine derartige Videokamera weist den Vorteil auf, daß sie mit einem Mikrorechner zusammenwirken kann. Es ist aber auch möglich, eine im Handel erhältliche CCD-Matrix oder eine
CID-Matrix aus Fotodetektorelementen zu verwenden.
Eine derartige Ausführungsform wird vorzugsweise
dann gewählt, wenn es auf Einfachheit und Zuverlässigkeit ankommt. Bei ihr sind auch die Kosten
und die Größe des Fingerabdrucksensors reduziert.

Die dargestellte Lichteinkopplungsmethode ist sehr
wirkungsvoll. Es ist nicht erforderlich, daß die
Randflächen 44, 46 der Kontaktplatte 14 zwecks
Erreichung eines guten Kontrastes im Fingerabdruckbild glatt sein müssen.

Gemäß den Figuren 2 bis 5 können auch Gitterkoppler
zum Einkoppeln von Licht in die dünne Kontaktplatte
14 verwendet werden. Derartige Koppler werden in
Verbindung mit dünnen Filmen benutzt (Proc. IEEE,
Vol. 62, Nr. 8, August 1974, S. 1044-1060, Figur 8).

Aus den Figuren 2 und 3 geht ein Amplitudengitter
hervor. Die Oberseite der Kontaktplatte 14 ist mit
einem Gitter bedeckt, welches aus einer Vielzahl
undurchsichtiger, parallel und eng nebeneinander
angeordneter Streifen gebildet ist. Das Licht tritt
in den Lichtwellenleiter bzw. die Kontaktplatte
14 durch Beugung an den Gitterstreifen 50 ein.

Gemäß den Figuren 4 und 5 können auch Phasengitter
verwendet werden. Phasengitter werden durch eine
gewisse Struktur in der Oberfläche eines transparenten Materials gebildet. In den Ausführungsformen gemäß
den Figuren 4 und 5 sind parallele Furchen und Rippen
52, 54 in der Lichtaufnahmefläche 20 der Kontaktplatte 14 ausgebildet. In der Ausführungsform nach Figur 4 weisen die Rippen 52 ein rechteckförmiges Profil auf, während in der Ausführungsform gemäß Figur 5 die Rippen ein krummlinig begrenztes Profil aufweisen.

Aus den Figuren 6 bis 8 geht hervor, daß ein Fingerabdrucksensor mehr als einen Lichtkoppler aufweisen kann. Durch mehrere Koppler kann eine gleichmäßige Lichtverteilung in der Kontaktplatte 14 erreicht werden, wenn die Kontaktfläche nicht von dem Finger 2 berührt wird.

Der in Figur 6 dargestellte Fingerabdrucksensor weist auf jedem Randbereich der Kontaktplatte 14 einen Prismenkoppler 12 bzw. 62 auf. Eine Lichtstreuung an den Rändern 44 und 46 hat deshalb keinen großen Einfluß auf die Qualität des Fingerabdruckbildes. Die Spalte zwischen der Kontaktplatte 14 und den Prismen 12 bzw. 62 sind mit 16 bzw. 66 bezeichnet.

In der Ausführungsform gemäß Figur 7 sind vier Lichtkoppler 70, 72, 74 und 75 auf einer rechteckförmigen Kontaktplatte 14 verwendet. Die Pfeile zeigen die Richtung des von vier nicht dargestellten Lichtquellen an. Die Lichtkoppler 70 bis 76 können Gitter und/oder Prismen sein. Die Kontaktfläche ist wiederum mit 20 bezeichnet.

Der in Figur 8 dargestellte Fingerabdrucksensor weist acht Lichtkoppler 81 bis 88 auf, die in einem regelmäßigen Achteck angeordnet sind. Demgemäß wird in die Kontaktplatte 14 Licht aus acht verschiedenen Richtungen eingestrahlt. Die Ausbreitungsrichtungen des Lichts sind durch acht Pfeile angedeutet, die ins Zentrum der Kontaktfläche 20 zeigen.

8 Patentansprüche
8 Figuren

0045916

-13. VPA 80 P 8234

Patentansprüche

1. Fingerabdrucksensor zum Erzeugen eines dem topografischen Relief eines zu untersuchenden Fingers (2) entsprechenden Ausgangssignals (a), g e k e n n - z e i c h n e t   d u r c h

a) eine aus einem transparenten, elastischen Material gebildete Kontaktplatte (14), die eine erste und eine dazu parallel angeordnete zweite Seite aufweist, wobei die erste Seite eine Lichtaufnahmefläche (18) zur Aufnahme von Licht und eine Kontaktfläche (20) zur Aufnahme eines durch den Finger (2) ausgeübten Kontaktdruckes aufweist, und die Kontaktfläche (20) ihre Struktur dem Fingerabdruckmuster des Fingers (2) entsprechend ändert, wenn der Finger auf sie aufgedrückt wird,

b) eine auf oder über der Lichtaufnahmefläche (18) der Kontaktplatte (14) vorgesehene Lichteinkoppelvorrichtung (12; 12, 62; 50; 52; 54;70, 72, 74, 76; 81 bis 88) zum Einkoppeln von Licht durch die Lichtaufnahmefläche (18) in die Kontaktplatte (14),

c) ein an die zweite Seite der Kontaktplatte (14) angrenzendes transparentes Medium (22), dessen Brechunsindex kleiner ist als der Brechungsindex der Kontaktplatte (14),

d) eine Lichtquelle (4), die der Lichteinkoppelvorrichtung Licht derart zusendet, daß das durch die Lichteinkoppelvorrichtung in die Kontaktplatte (14) eingedrungene Licht in dieser Platte total reflektiert wird und dadurch auch die Kontaktfläche (20) trifft, wodurch in Gegenwart des Fingers (2) die Kontaktfläche (20) einen reflektierten Lichtstrahl (30) erzeugt, der die zweite Seite der Sensorplatte und das transparente Medium (22) durchstrahlt und der mit dem Finger-

-14-　　　VPA 80 P 8234

abdruckmuster des Fingers (2) moduliert ist,

e) eine Fotodetektoreinrichtung (36, 38) mit einem lichtempfindlichen Bereich (38) zum Messen der Verteilung des auf diesen Bereich auftreffenden Lichts,

f) eine optische Einrichtung (32), welche den von der Kontaktfläche (20) reflektierten Lichtstrahl (30) dem lichtempfindlichen Bereich (36) der Fotodetektoreinrichtung (36, 38) zuführt,

g) eine zwischen der optischen Einrichtung (32) und dem lichtempfindlichen Bereich (36) angeordnete Blendeneinrichtung (43), die den reflektierten Lichtstrahl (30) teilweise ausblendet, und

h) durch eine mit der Fotodetektoreinrichtung (36, 38) verbundene Ausgabeeinrichtung (40, 42), die das elektrische Ausgangssignal (a) erzeugt und ausgibt.

2. Fingerabdrucksensor nach Anspruch 1, d a d u r c h   g e k e n n z e i c h n e t , daß die Lichteinkoppelvorrichtung ein Prisma (12, 62) mit wenigstens einer planen Seitenfläche aufweist, die durch einen schmalen, ein transparentes Medium niedrigen Brechungsindexes enthaltenden Spalt (16) von der Lichtaufnahmefläche (18) getrennt ist, und /oder einen der Lichtaufnahmefläche (18) zugeordneten oder damit verbundenen Gitterkoppler (50; 52; 54; 70, 72, 74, 76; 81 bis 88) zum Einkoppeln von Licht in die Kontaktplatte (14) aufweist.

3. Fingerabdrucksensor nach Anspruch 2, d a d u r c h   g e k e n n z e i c h n e t , daß der Spalt (16, 66) Luft enthält.

4. Fingerabdrucksensor nach Anspruch 2 oder 3, d a d u r c h   g e k e n n z e i c h n e t , daß der Spalt (16, 66) annähernd 100 nm breit ist.

5. Fingerabdrucksensor nach Anspruch 2, d a d u r c h
g e k e n n z e i c h n e t , daß der Gitterkoppler
ein Phasengitter (52; 54) aufweist.

6. Fingerabdrucksensor nach einem der vorhergehenden
Ansprüche, d a d u r c h   g e k e n n z e i c h -
n e t , daß das transparente Medium (22) mit dem
vergleichsweise niedrigeren Brechungsindex ein an
die zweite Seite der Kontaktplatte (14) angebrachtes
Substrat ist.

7. Fingerabdrucksensor nach einem der vorhergehenden
Ansprüche, d a d u r c h   g e k e n n z e i c h -
n e t , daß eine weitere optische Einrichtung (6)
so angeordnet ist, daß sie Licht aus der Lichtquelle
(4) der Lichtaufnahmefläche (18) zuführt, und daß
diese weitere optische Einrichtung (6) eine Kollimatorlinse (8) aufweist.

8. Fingerabdrucksensor nach einem der vorhergehenden
Ansprüche, d a d u r c h   g e k e n n z e i c h -
n e t , daß die optische Einrichtung (32) wenigstens
eine Abbildungslinse (34) aufweist, welche die
Kontaktfläche (20) auf den lichtempfindlichen
Bereich (36) der Fotodetektoreinrichtung (36, 38)
abbildet, und daß die Blendeneinrichtung (43)
aus einer Schneide besteht, die in einer zwischen
dem transparenten Medium (22) und dem lichtempfindlichen Bereich (36) liegenden Brennebene der Abbildungslinse (34) angeordnet ist.

FIG. 1

0045916

2/2

FIG. 2

FIG. 3

FIG. 4

FIG. 5

FIG. 6

FIG. 7

FIG. 8

Europäisches
Patentamt

## EUROPÄISCHER RECHERCHENBERICHT

| | EINSCHLÄGIGE DOKUMENTE | | KLASSIFIKATION DER ANMELDUNG (Int Cl ³) |
|---|---|---|---|
| Kategorie | Kennzeichnung des Dokuments mit Angabe, soweit erforderlich, der maßgeblichen Teile | betrifft Anspruch | A 61 B 5/10 |
| | FR - A - 2 407 530 (M. SCHILLER)<br>* Seite 1, Zeile 38 - Seite 2, Zeile 28; Abbildungen; Seite 4, Zeile 26 - Seite 5, Zeile 38 *<br>-- | 1,7 | |
| D | US - A - 4 120 585 (V.A. DE PALMA)<br>* Zusammenfassung; Abbildungen *<br>-- | 1,7 | |
| | US - A - 3 619 060 (J.E. JOHNSTON)<br>* Zusammenfassung; Abbildung 2; Spalte 3, Zeile 52 - Spalte 4, Zeile 19 *<br>-- | 1,6,7 | **RECHERCHIERTE SACHGEBIETE (Int Cl.³)**<br><br>A 61 B 5/10<br>G 07 C 9/00<br>11/00 |
| | US - A - 3 864 042 (S.R. LEVENTHAL)<br>* Zusammenfassung; Abbildungen; Spalte 3, Zeile 66 - Spalte 4, Zeile 7 *<br>-- | 1-3,7 | |
| A | US - A - 3 407 715 (C.W. McCUTCHEN)<br>* Zusammenfassung; Abbildungen 1,3; Spalte 3, Zeilen 34-41 *<br>---- | 1,2 | |

**KATEGORIE DER GENANNTEN DOKUMENTE**

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

Der vorliegende Recherchenbericht wurde für alle Patentansprüche erstellt.

| Recherchenort | Abschlußdatum der Recherche | Prüfer |
|---|---|---|
| Den Haag | 19-11-1981 | DAVID |

EPA form 1503.1   06.78